# EUROPEAN PATENT APPLICATION

(11) **EP 2 524 966 A1**
(43) Date of publication of application: **21.11.2012**
(21) Application number: 11166628.5
(22) Date of filing: 18.05.2011
(51) Int. Cl.: C12Q 1/68

(54) **Molecular analysis of tuberculosis**

(71) Applicant: Rheinische Friedrich-Wilhelms-Universität Bonn, 53113 Bonn (DE)
(72) Inventor: Schultze, Joachim, 53639 Königswinter (DE); Hofmann, Andrea, 53111 Bonn (DE); Staratschek-Jox, Andrea, 50733 Köln (DE)
(74) Representative: von Kreisler Selting Werner

(57) **Abstract**

The present invention provides a method for molecular analysis of active tuberculosis (in short only 'TB') based on the abundance of particular RNAs from blood samples, as well as diagnostic tools such as kits and arrays suitable for such method.

## Description

The present invention provides a method for molecular analysis of active tuberculosis (herein shortly only 'TB') based on the abundance of particular RNAs from blood samples, as well as diagnostic tools such as kits and arrays suitable for such method.

### Background of the Invention

With the initial introduction of DNA microarray analysis for cancer diagnostics in the late 1990s (T.R. Golub et al., Science 286:531-537 (1999)), a rush towards diagnostic, prognostic and even predictive gene signatures was initiated (J.A. Ludwig and J.N. Weinstein, Nat. Rev. Cancer 5:845-856 (2005); A. Rosenwald et al., New England J. Med. 346:1937-1947 (2002); E.J. Yeoh et al., Cancer Cell 1:133-143 (2002); L.J. van 't Veer et al., Nature 415:530-536 (2002); D.G. Beer et al., Nat. Med. 8: 816-824 (2002); A. Bhattacharjee et al., Proc. Natl. Acad. Sci. USA 98: 13790-13795 (2001); S. Ramaswamy et al., Nat. Genet. 33: 49-54 (2003); S.L. Pomeroy et al., Nature 415: 436-442 (2002)). Not much later similar strategies were applied to other areas of medical sciences, e.g. infectious diseases (M.P. Berry et al., Nature 466: 973-977 (2010)). At the same time, there was a tremendous surge for novel analytical tools and mathematical algorithms to be utilized for the analysis of high throughput data for diagnostic purposes (M.D. Radmacher et al., J Comput Biol 9: 505-511(2002); A.M. Glas et al., BMC Genomics 7:278 (2006); Q. Liu et al., PLoS One 4: e8250 (2009); T. Reme et al., BMC Bioinformatics 9:16(2008); R.M: Parry et al., Pharmacogenomics J. 10:292-309 (2010)). Irrespective of these significant advances, the number of gene signatures that have entered clinical practice is alarmingly small (FDA, FDA Clears Breast Cancer Specific Molecular Prognostic Test (2007) and F.M. Goodsaid et al., Nat Rev Drug Discov 9:435-445 (2010)). More futile, there have been serious concerns about the validity of several landmark studies in gene signature development (S. Michiels et al., Lancet 365:488-492 (2005)) if not about the approach in general (S. Michiels et al., Lancet 365:488-492 (2005); J.P. Ioannidis et al., Nat Genet 41: 149-155 (2009); A. Dupuy A, R.M. Simon, J Natl Cancer Inst 99: 147-157(2007)). As an important result of these concerns the MicroArray Quality Control (MAQC-I) project was successfully installed demonstrating that the technology itself is reliable and reproducible (L. Shi et al., Nat Biotechnol 24:1151-1161(2006); L. Shi et al., Curr Opin Biotechnol 19:10-18 (2008)). More recently, concerns about overoptimistic data presentation in clinical gene signature studies could be eased by improving the analytical processes used within these pilot studies (X. Fan et al., doi: 10.1158/1078-0432.CCR-09-1815). In addition, the MAQC-II study set the framework for further development of microarray-based predictive models (L. Shi et al., Nat Biotechnol 28: 827-838(2010); S. Dudoit et al., J. Am. Stat. Assoc. 97, 77-87 (2002). Several important points are derived from this large consortium effort. Most important, model prediction performance is largely (biological) endpoint dependent, probably the most critical finding supporting further development of gene signature technology. Further, internal validation performance from well-implemented, unbiased cross-validation shows a high degree of concordance with external validation performance. Nevertheless, external validation is a critical feature for signature development. Formerly questioned by others [L. Ein-Dor et al., Bioinformatics 21: 171-178 (2005); L. Ein-Dor et al., Proc Natl Acad Sci U S A 103: 5923-5928 (2006)) the MAQC-II study also clearly established that many classifiers with similar performance can be developed from a given data set. Not surprising, proficiency of investigators and good modeling practice are leading to improved results (L. Shi et al., Nat Biotechnol 28: 827-838 (2010)).

Since these comprehensive findings strongly suggest to quickly develop high throughput gene expression data into diagnostic tests we sought to address several unresolved issues. First, to better judge the validity of small pilot trials we developed a validation approach combined with randomized permutation ("10.000 clinical trial simulation"). This approach can also estimate overall best test performance. Utilizing these approaches we introduce a high-performance test for diagnosis of patients with active tuberculosis.

### Summary of the Invention

Increasing data support the notion that biological high throughput data will transform molecular diagnostics of many diseases including cancer and infection. Among the most advanced approaches are gene signatures based on gene expression profiling of diseased tissue or peripheral blood. However, despite the enormous number of studies performed translation of gene signatures into clinical use has been very limited and continues to be tremendously difficult. Here, we introduce simulation approaches to significantly improve and accelerate the development of gene signature-based diagnostic biomarkers as exemplified for tuberculosis. In addition to current approaches determining optimal classifiers within a defined study setting (training and validation set), the overall study setting (n>10.000) was permutated thereby simulating the performance range (sensitivities, specificities, AUC) of potential disease classifiers in other study settings. With these significant improvements we establish an exceedingly robust and clinically applicable gene signature for the diagnosis of tuberculosis. The invention thus provides methods and kits for diagnosing, detecting, and screening for of tuberculosis (TB) using blood-based gene expression samples (GEP). Particularly, the invention provides for preparing RNA expression profiles of patient blood samples, the RNA expression profiles being indicative of the presence or absence of TB. The invention further provides for evaluating the patient RNA expression profiles for the presence or absence of one or more RNA expression signatures that are indicative ofTB.

The conclusion whether the patient has TB or not may comprise, in a preferred embodiment of the method, classifying the sample as being from a healthy individual or from an individual having TB based on the specific difference of the abundance of the at least 4 RNAs in healthy individuals versus the abundance of the at least 4 RNAs in individuals with TB (in a reference set). In the present method, a sample can be classified as being from a patient with TB or from a healthy individual without the necessity to run a reference sample of known origin (i.e. from a TB patient or a healthy individual) at the same time.

In a preferred embodiment the method of the invention is a method for the detection of TB in a human individual based on RNA obtained from a blood sample obtained from the individual, comprising:
determining the abundance of at least 4 RNAs in the sample that are chosen from the RNAs listed in Table 1, and
classifying the sample as being from a healthy individual or from an individual having TB based on the specific difference of the abundance of the at least 4 RNAs in healthy individuals versus the abundance of the at least 4 RNAs in individuals with TB. Particularly preferred for this method is that the abundance of at least 6 RNAs, of at least 8 RNAs, of at least 10 RNAs, of at least 12 RNAs, or of at least 14 RNAs listed in Table 1 is determined. It is further preferred to determine the highest ranked RNAs of Table 1 in the method of the invention.

In one aspect, the invention provides a method for preparing RNA expression profiles that are indicative of the presence or absence of TB. The RNA expression profiles are prepared from patient blood samples. The number of transcripts in the RNA expression profile may be selected so as to offer a convenient and cost effective means for screening samples for the presence or absence of TB with high sensitivity and high specificity. Generally, the RNA expression profile includes the expression level or "abundance" of from 4 to about 3000 transcripts. In certain embodiments, the expression profile includes the RNA levels of 2500 transcripts or less, 2000 transcripts or less, 1500 transcripts or less, 1000 transcripts or less, 500 transcripts or less, 250 transcripts or less, 100 transcripts of less, or 50 transcripts or less.

In such embodiments, the profile may contain the abundance or expression level of at least 4 RNAs that are indicative of the presence or absence of TB, and specifically, as selected from Table 1, or may contain the expression level of at least 6, at least 8, at least 12 or at least 14 RNAs selected from Table 1. Where larger profiles are desired, the profile may contain the expression level or abundance of at least about 60, at least 100, at least 150, or 200 RNAs that are indicative of the presence or absence of TB, and such RNAs may be selected from Table 1.

Such RNA expression profiles in accordance with this aspect may be evaluated for the presence or absence of an RNA expression signature indicative of TB. Generally, the sequential addition of transcripts from Table 1 to the expression profile provides for higher sensitivity and/or specificity for the detection of TB. For example, the area under the ROC curve (AUC) may be at least at least 0.8, or at least 0.82, or at least 0.85 or at least 0.9. The AUC is a quantitative parameter for the clinical utility (specificity and sensitivity) of the detection method described herein. An AUC of 1.0 refers to a sensitivity and specificity of 100 %.

Alternatively median Mathews Correlation Coefficient (MCC) may be at least at least 0.8, or at least 0.82, or at least 0.85 or at least 0.9. Again, the MCC is a quantitative parameter for the clinical utility (specificity and sensitivity) of the detection method described herein. An MCC of 1.0 refers to a sensitivity and specificity of 100 %.

In a second aspect, the invention provides a method for detecting, diagnosing, or screening for TB. In this aspect, the method comprises preparing an RNA expression profile by measuring the abundance of at least 4, at least 6, at least 8, or at least 12, or at least 14 RNAs in a patient blood sample, where the abundance of such RNAs are indicative of the presence or absence of TB. The RNAs may be selected from the RNAs listed in Table 1. The method further comprises evaluating the profile for the presence or absence of an RNA expression signature indicative of TB, to thereby conclude whether the patient has or does not have TB. The method generally provides a sensitivity for the detection of TB of at least about 70 %, while providing a specificity of at least about 70 %.

In various embodiments, the method comprises determining the abundance of at least 4 RNAs, at least 60 RNAs, at least 100 RNAs, at least 150, or of at least 300 RNAs chosen from the RNAs listed in Table 1, and classifying the sample as being indicative of TB, or not being indicative of TB.

In other aspects, the invention provides kits and custom arrays for preparing the gene expression profiles, and for determining the presence or absence of TB.

### Short Description of the Figures

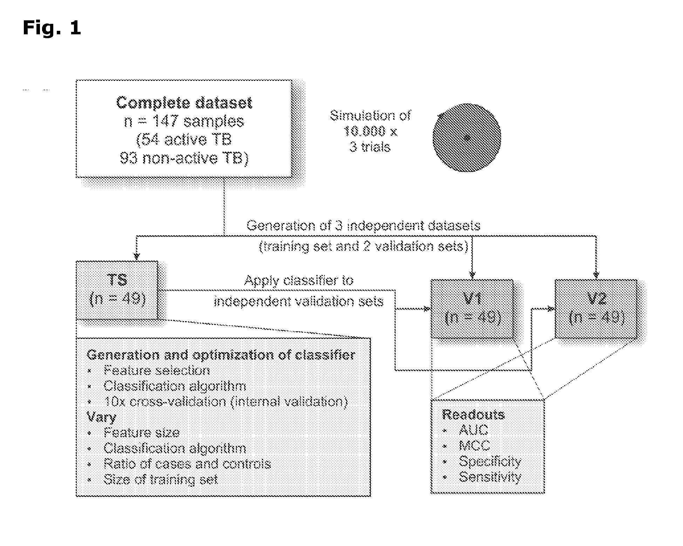

Figure 1: Development of a classifier to molecularly diagnose active TB. (A) Schema of approach to define classifiers. A dataset containing a total of 149 samples was used.Within these 149 samples, 54 cases of active TB were included, the other samples were called non-TB or control samples. The 150 samples were evenly split into three independent cohorts of 49 samples, called training set (TS) and validation set 1 (V1) and validation set 2 (V2). The classifier was built in TS and applied to V1 and V2. Classifier performance is only shown for external validation in V1 and V2. Classifiers were build according to a combined approach of 1) feature selection, 2) application of a classifier algorithm, and 3) 10-fold cross-validation (internal validation). Influence of 1) feature size, 2) classification algorithm, 3) ratio of cases and controls in the TS, and 4) the size of the TS were assessed by varying the respective parameters. As readout we assessed AUC and MCC, . The process of generating three independent sets of data out of the 150 samples was performed 10,000 times and termed 'trial simulation approach' (TSA).

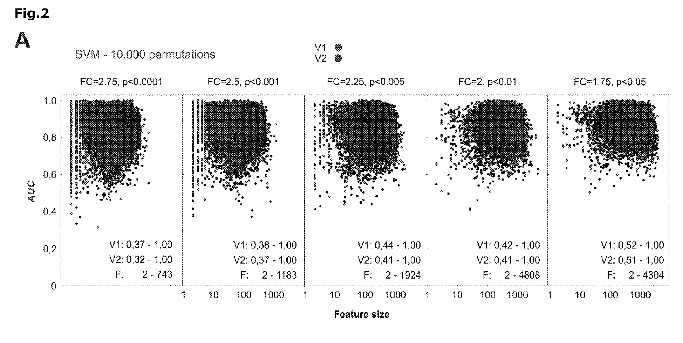

Figure 2: Correlation of feature size and feature selection with classifier performance. The number of features in each individual classifier of a total of 10.000 classifiers is plotted against the AUC of the respective classifier. For each level of filter settings (a total of five different levels of filter settings) the data are plotted separately. It can be clearly seen that the variation in AUC is reduced with higher feature sizes.

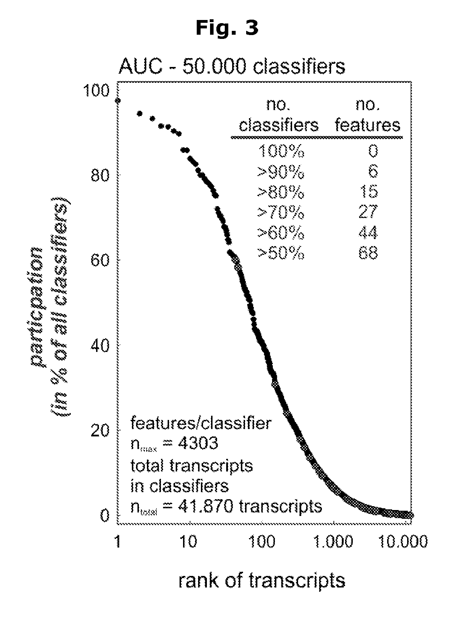

Figure 3: For each transcript interrogated on the array, its participation in any of the 60.000 classifier (6 levels of filtering) were calculated and ranked. If a transcript was part of at least 1 classifier, its participation frequency was plotted.

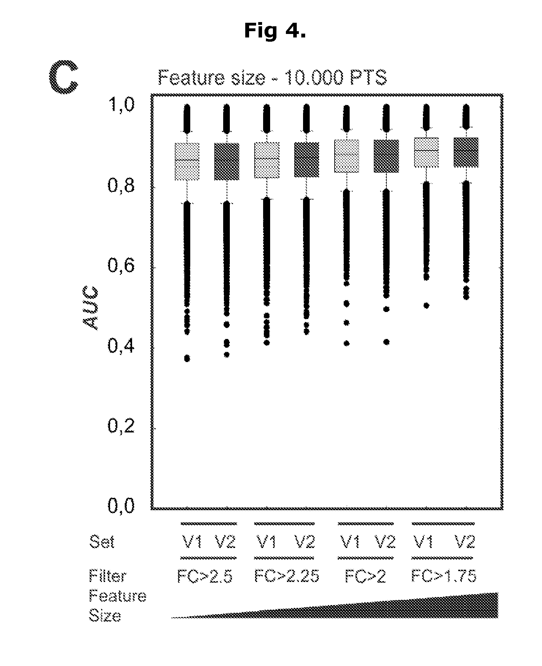

Figure 4: The influence of feature size on classifier performance was assessed again using 10,000 independent TSA settings. Filter based on differentially expressed genes (combined fold change, p-value filter, here abbreviated by FC only) were used. The larger the FC value the smaller the number of transcripts per classifier. Shown is again classifier performance in V1 and V2 independently. Data are shown in an integrated format shown as a boxplot (mean, 25 to 75 percentiles, standard deviation and outliers).

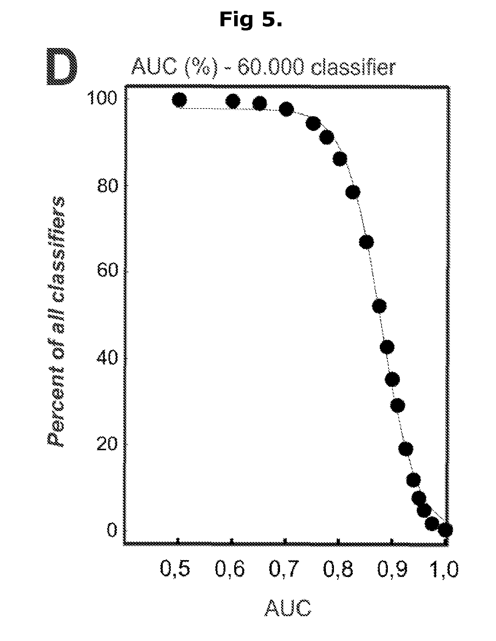

Figure 5: Classifier performance (here AUC) was plotted against the frequency of classifiers reaching a certain AUC level.

### Detailed Description of the invention

The invention provides methods and kits for screening, diagnosing, and detecting active TB in human patients (subjects). A synonym for a patient with active TB is "TB-case" or simply "case."

As disclosed herein, the present invention provides methods and kits for screening patient samples for those that are positive for TB, e.g., in the absence of surgery or any other diagnostic procedure.

The invention relates to the determination of the abundance of RNAs to detect a TB in a human subject, wherein the determination of the abundance is based on RNA obtained (or isolated) from whole blood of the subject or from blood cells of the subject.

In various aspects, the invention involves preparing an RNA expression profile from a patient sample. The method may comprise isolating RNA from whole blood, and detecting the abundance or relative abundance of selected transcripts. The "RNAs" may be defined by reference to an expressed gene, or by reference to a transcript, or by reference to a particular oligonucleotide probe for detecting the RNA (or cDNA derived therefrom), each of which is listed in Table 1 for 300 RNAs that are indicative of the presence or absence of TB.

The number of transcripts in the RNA expression profile may be selected so as to offer a convenient and cost effective means for screening samples for the presence or absence of TB with high sensitivity and high specificity. For example, the RNA expression profile may include the expression level or "abundance" of from 4 to about 3000 transcripts. In certain embodiments, the expression profile includes the RNA levels of 2500 transcripts or less, 2000 transcripts or less, 1500 transcripts or less, 1000 transcripts or less, 500 transcripts or less, 250 transcripts or less, 200 transcripts of less, 100 transcripts of less, or 50 transcripts or less. Such profiles may be prepared, for example, using custom microarrays or multiplex gene expression assays as described in detail herein.

Such RNA expression profiles in accordance with this aspect may be evaluated for the presence or absence of an RNA expression signature indicative of TB. Generally, the sequential addition of transcripts from Table 1 to the expression profile provides for higher sensitivity and/or specificity for the detection of TB, as indicated by the AUC. A clinical utility is reached if the AUC is at least 0.8.

The inventors have surprisingly found that an AUC of 0.8 is reached if and only if at least 4 RNAs are measured that are chosen from the RNAs listed in Table 1. In other words, measuring 4 RNAs is necessary and sufficient for the detection of TB in a human subject based on RNA from a blood sample obtained from said subject by measuring the abundance of at least 4 RNAs in the sample, that are chosen from the RNAs listed in Table 1, and concluding based on the measured abundance whether the subject has TB or not. An analysis of 1, 2 or 3 RNAs chosen from the RNAs listed in Table 1, however, does not allow for this detection.

For example, the area under the ROC curve (AUC) may be at least 0.8, or at least 0.82, or at least 0.85 or at least 0.9. The AUC is a quantitative parameter for the clinical utility (specificity and sensitivity) of the detection method described herein. An AUC of 1.0 refers to a sensitivity and specificity of 100 %. In such embodiments, the profile may contain the expression level of at least 4 RNAs that are indicative of the presence or absence of TB, and specifically, as selected from Table 1, or may contain the expression level of at least 6, 8, 10, 12 or 14 RNAs selected from Table 1. Where larger profiles are desired, the profile may contain the expression level or abundance of at least 60, 100, 200, or 300 RNAs that are indicative of the presence or absence of TB, and such RNAs may be (at least in part) selected from Table 1. Such RNAs may be defined by gene, or by transcript ID, or by probe ID.

The identities of genes and/or transcripts that make up, or are included in exemplary expression profiles are disclosed in Table 1. As shown herein, profiles selected from the RNAs of Table 1 support the detection of TB with high sensitivity and high specificity.

Thus, in various embodiments, the abundance of at least 4, at least 6, at least 8, at least 10, at least 12, at least 14, at least 60, at least 100, at least 150, or at least 300 distinct RNAs are measured, in order to arrive at a reliable diagnosis of TB. The set of RNAs may comprise, consist essentially of, or consist of, a set or subset of RNAs exemplified in Table 1. The term "consists essentially of" in this context allows for the expression level of additional transcripts to be determined that are not differentially expressed in TB subjects, and which may therefore be used as positive or negative expression level controls or for normalization of expression levels between samples.

Such RNA expression profiles may be evaluated for the presence or absence of an RNA expression signature indicative of TB. Generally, the sequential addition of transcripts from Table 1 to the expression profile provides for higher sensitivity and/or specificity and stability (i.e. independence from the sample analyzed) for the detection of TB. For example, the sensitivity and specificity of the methods provided herein may be equivalent to an area under the ROC curve (AUC) of at least at least 0.8, or at least 0.82, at least 0.85, or of at least 0.9.

The present invention provides an in-vitro diagnostic test system (IVD) that is trained (as described further below) for the detection of a TB. For example, in order to determine whether a patient has TB, reference RNA abundance values for TB positive and negative samples are determined. The RNAs can be quantitatively measured on an adequate set of training samples comprising cases and controls, and with adequate clinical information on active TB status, applying adequate quality control measures, and on an adequate set of test samples, for which the detection is yet to be made. With such quantitative values for the RNAs and the clinical data for the training samples, a classifier can be trained and applied to the test samples to calculate the probability of the presence or non-presence of active TB. Therefore, in one embodiment of the present method, a sample can be classified as being from a patient with TB or from a healthy individual without the necessity to run a reference sample of known origin (i.e. from a TB patient or a healthy individual) at the same time.

Various classification schemes are known for classifying samples between two or more classes or groups, and these include, without limitation: Naïve Bayes, Support Vector Machines, Nearest Neighbors, Decision Trees, Logistics, Articifial Beural Networks, and Rule-based schemes. In addition, the predictions from multiple models can be combined to generate an overall prediction. Thus, a classification algorithm or "class predictor" may be constructed to classify samples. The process for preparing a suitable class predictor is reviewed in R. Simon, Diagnostic and prognostic prediction using gene expression profiles in high-dimensional microarray data, British Journal of Cancer (2003) 89, 1599-1604, which review is hereby incorporated by reference.

In this context, the invention teaches an in-vitro diagnostic test system (IVD) that is trained in the detection of a TB referred to above, comprising at least 4 RNAs, which can be quantitatively measured on an adequate set of training samples comprising cases and controls, with adequate clinical information on leukemia status, applying adequate quality control measures, and on an adequate set of test samples, for which the detection yet has to be made. Given the quantitative values for the RNAs and the clinical data for the training samples, a classifier can be trained and applied to the test samples to calculate the probability of the presence or absence of the TB.

The present invention provides methods for detecting, diagnosing, or screening for TB in a human subject with a high sensitivity and specificity. Specifically, the sensitivity of the methods provided herein is equivalent to an area under the ROC curve (AUC) of at least at least 0.8, or at least 0.82, at least 0.85, of or at least 0.9.

Without wishing to be bound by any particular theory, the above finding may be due to the fact that an organism such as a human systemically reacts to the development of an active TB by altering the expression levels of genes in different pathways. Although the change in expression (abundance) might be small for each gene in a particular signature, measuring a set of at least 4 genes, preferably even larger numbers such as 6, 8, 10, 12, 14, 100, 150, 300 or even more RNAs, for example at least 5, at least 8, at least 120, at least 160 RNAs at the same time allows for the detection of TB in a human with high sensitivity and high specificity.

In this context, a RNA obtained from a subject's blood sample, i.e. a RNA biomarker, is a RNA molecule with a particular base sequence whose presence within a blood sample from a human subject can be quantitatively measured. The measurement can be based on a part of the RNA molecule, namely a part of the RNA molecule that has a certain base sequence, which allows for its detection and thereby allows for the measurement of its abundance in a sample. The measurement can be by methods known in the art, for example analysis on a solid phase device, or in solution (for example, by RT-PCR). Probes for the particular RNAs can either be bought commercially, or designed based on the respective RNA sequence.

In the method of the invention, the abundance of several RNA molecules (e.g. mRNA or pre-spliced RNA, intron-lariat RNA, micro RNA, small nuclear RNA, or fragments thereof) is determined in a relative or an absolute manner, wherein an absolute measurement of RNA abundance is preferred. The RNA abundance is, if applicable, compared with that of other individuals, or with multivariate quantitative thresholds.

The determination of the abundance of the RNAs described herein is performed from blood samples using quantitative methods. In particular, RNA is isolated from a blood sample obtained from a human subject that is to undergo TB testing, i.e. a person with fever, coughing and/or weakness, or a person with a clinical history of being in contact with Tb patients. Although the examples described herein use microarray-based methods, the invention is not limited thereto. For example, RNA abundance can be measured by in situ hybridization, amplification assays such as the polymerase chain reaction (PCR), sequencing, or microarray-based methods. Other methods that can be used include polymerase-based assays, such as RT-PCR (e.g., TAQMAN), hybridization-based assays, such as DNA microarray analysis, as well as direct mRNA capture with branched DNA (QUANTIGENE) or HYBRID CAPTURE (DIGENE).

In certain embodiments, the invention employs a microarray. A "microarray" includes a specific set of probes, such as oligonucleotides and/or cDNAs (*e.g.*, expressed sequence tags, "tests") corresponding in whole or in part, and/or continuously or discontinuously, to regions of RNAs that can be extracted from a blood sample of a human subject. The probes are bound to a solid support. The support may be selected from beads (magnetic, paramagnetic, etc.), glass slides, and silicon wafers. The probes can correspond in sequence to the RNAs of the invention such that hybridization between the RNA from the subject sample (or cDNA derived therefrom) and the probe occurs. In the microarray embodiments, the sample RNA can optionally be amplified before hybridization to the microarrays. Prior to hybridization, the samples RNA is fluorescently labeled. Upon hybridization to the array and excitation at the appropriate wavelength, fluorescence emission is quantified. Fluorescence emission for each particular RNA is directly correlated with the amount of the particular RNA in the samples. The signal can be detected and together with its locution on the support can be used to determine which probe hybridized with RNA from the subject's blood sample.

Accordingly, in certain aspects, the invention is directed to a kit or microarray for detecting the level of expression or abundance of RNAs in the subject's blood samples, where this "profile" allows for the conclusion of whether the subject has TB or not (at a level of accuracy described herein). In another aspect, the invention relates to a probe set that allows for the detection of the RNAs associated with TB. If these particular RNAs are present in a sample, they (or corresponding cDNA) will hybridize with their respective probe (i.e, a complementary nucleic acid sequence), which will yield a detectable signal. Probes are designed to minimize cross reactivity and false positives.

Thus, the invention in certain aspects provides a microarray, which generally comprises a solid support and a set of oligonucleotide probes. The set of probes generally contains from 4 to about 3,000 probes, including at least 4 probes deduced from Table 1. In certain embodiments, the set contains 2000 probes or less, or 1000 probes or less, 500 probes or less, 200 probes or less, or 100 probes or less.

The conclusion whether the subject has TB or not is preferably reached on the basis of a classification algorithm, which can be developed using e.g. a random forest method, a support vector machine (SVM), a K-nearest neighbor method (K-NN), such as a 3-nearest neighbor method (3-NN), a linear discrimination analysis (LDA), or a prediction analysis for microarrays (PAM), as known in the art.

Preferably, F-statistics (ANOVA) is used to identify specific difference of the abundance of the at least 4 RNAs in healthy individuals versus the abundance of the at least 4 RNAs in individuals with TB.

"Sensitivity" (S⁺ or true positive fraction (TPF)) refers to the count of positive test results among all true positive disease states divided by the count of all true positive disease states.

"Specificity" (S- or true negative fraction (TNF)) refers to the count of negative test results among all true negative disease states divided by the count of all true negative disease states.

"Correct Classification Rate" (CCR or true fraction (TF)) refers to the sum of the count of positive test results among all true positive disease states and count of negative test results among all true negative disease states divided by all the sum of all cases. The measures S⁺, S⁻, and CCR address the question: To what degree does the test reflect the true disease state?

"Positive Predictive Value" (PV⁺ or PPV) refers to the count of true positive disease states among all positive test results dived by the count of all positive test results.

"Negative Predictive Value" (PV- or NPV) refers to the count of true negative disease states among all negative test results dived by the count of all negative test results. The predictive values address the question: How likely is the disease given the test results?

The preferred RNA molecules that can be used in combinations described herein for diagnosing and detecting TB in a subject according to the invention can be found in Table 1. The inventors have shown that the selection of at least 4 or more RNAs of the markers listed in Table 1 can be used to diagnose or detect TB in a subject using a blood sample from that subject. The RNA molecules that can be used for detecting, screening and diagnosing TB are selected from the RNAs provided in Table 1.

Specifically, the method of the invention comprises at least the following steps: measuring the abundance of at least 4 RNAs (preferably 9 RNAs or 10 RNAs) in the sample, that are chosen from the RNAs listed in table 3, and concluding, based on the measured abundance, whether the subject has TB or not. Measuring the abundance of RNAs may comprise isolating RNA from blood samples as described, and hybridizing the RNA or cDNA prepared therefrom to a microarray. Alternatively, other methods for determining RNA levels may be employed.

In a preferred embodiment of the invention as mentioned herein, the abundance of at least 4 RNAs (preferably 6, 8, 10, or 12 RNAs) in the sample is measured, wherein the at least 4 RNAs are chosen from the RNAs listed in Table 1. Examples for sets of 4 RNAs that can be measured together, i.e. sequentially or preferably simultaneously, to detect TB in a human subject are shown in Table 1.

An example for a set of 300 RNAs of which the abundance can be measured in the method of the invention is listed in Table 1.

The wording "at least a number of RNAs" refers to a minimum number of RNAs that are measured. It is possible to use up to 10,000 or 20,000 genes in the invention, a fraction of which can be RNAs listed in Table 1. In preferred embodiments of the invention, abundance of up to 5.000, 2.500, 2.000, 1,000, 500, 250, 100, 80, 70, 60, 50, 40, 30, 20, 10, 5,4, 3, 2, or 1 RNA of randomly chosen RNAs that are not listed in table 1 is measured in addition to RNAs of table 1 (or subsets thereof).

In a preferred embodiment, only RNAs that are mentioned in Table 1 are measured.

The expression profile or abundance of RNA markers for TB, for example the at least 4 RNAs described above, (or more RNAs as disclosed above and herein), is determined preferably by measuring the quantity of the transcribed RNA of the marker gene. This quantity of the mRNA of the marker gene can be determined for example through chip technology (microarray), (RT-) PCR (for example also on fixated material), Northern hybridization, dot-blotting, sequencing, or in situ hybridization.

The microarray technology, which is most preferred, allows for the simultaneous measurement of RNA abundance of up to many thousand RNAs and is therefore an important tool for determining differential expression (or differences in RNA abundance), in particular between two biological samples or groups of biological samples. In order to apply the microarray technology, the RNAs of the sample need to be amplified and labeled and the hybridization and detection procedure can be performed as known to a person of skill in the art.

As will be understood by those of ordinary skill in the art, the analysis can also be performed through single reverse transcriptase--PCR, competitive PCR, real time PCR, differential display RT-PCR, Northern blot analysis, sequencing, and other related methods. In general, the larger the number of markers is that are to be measured, the more preferred is the use of the microarray technology. However, multiplex PCR, for example, real time multiplex PCR is known in the art and is amenable for use with the present invention, in order to detect the presence of 2 or more genes or RNAs simultaneously.

The RNA whose abundance is measured in the method of the invention can be mRNA, cDNA, unspliced RNA, or its fragments. Measurements can be performed using the complementary DNA (cDNA) or complementary RNA (cRNA), which is produced on the basis of the RNA to be analyzed, e.g. using microarrays. A great number of different arrays as well as their manufacture are known to a person of skill in the art and are described for example in the U.S. Patent Nos.

Preferably the decision whether the subject has TB comprises the step of training a classification algorithm on an adequate training set of cases and controls and applying it to RNA abundance data that was experimentally determined based on the blood sample from the human subject to be diagnosed. The classification method can be a random forest method, a support vector machine (SVM), or a K-nearest neighbor method (K-NN), such as 3-NN.

For the development of a model that allows for the classification for a given set of biomarkers, such as RNAs, methods generally known to a person of skill in the art are sufficient, i.e., new algorithms need not be developed.

The major steps of such a model are:
1) condensation of the raw measurement data (for example combining probes of a microarray to probe set data, and/or normalizing measurement data against common controls);
2) training and applying a classifier (i.e. a mathematical model that generalizes properties of the different classes (active TB vs. healthy individual) from the training data and applies them to the test data resulting in a classification for each test sample.

For example, the raw data from microarray hybridizations can first be condensed with FARMS as shown by Hochreiter et al., Bioinformatics 22(8): 943-9(2006). Alternative methods for condensation such as Robust Multi-Array Analysis (RMA, GC-RMA, see Irizarry et al. Biostatistics. 4, 249-264 (2003) can be used. Similar to condensation, classification of the test data set through a support-vector-machine or other classification algorithms is known to a person of skill in the art, like for example classification and regression trees, penalized logistic regression, sparse linear discriminant analysis, Fisher linear discriminant analysis, K-nearest neighbors, shrunken centroids, and artificial neural networks (see W. Wapnik, The Nature of Statistical Learning Theory, Springer Verlag, New York, NY, USA, 1995; Berhard Scholkopf, Alex Smola: Learning with Kernels: Support Vector Machines, Regularization, Optimization, and Beyond, MIT Press, Cambridge, MA, 2002; S. Kotsiantis, Informatica J. 31:249-268 (2007)).

The key component of these classifier training and classification techniques is the choice of RNA biomarkers that are used as input to the classification algorithm. In a further aspect, the invention refers to the use of a method as described above and herein for the detection of TB in a human subject, based on RNA from a blood sample.

In a further aspect, the invention also refers to the use of a microarray for the detection of TB in a human subject based on RNA from a blood sample. According to the invention, such a use can comprise measuring the abundance of at least 4 RNAs (or more, as described above and herein) that are listed in Table 1. Accordingly, the microarray comprises at least 3 probes for measuring the abundance of the at least 3 RNAs. Commercially available microarrays, such as from Illumina or Affymetrix, may be used.

In another embodiment, the abundance of the at least 4 RNAs is measured by multiplex RT-PCR. In a further embodiment, the RT-PCR includes real time detection, e.g., with fluorescent probes such as Molecular beacons or TaqMan® probes.

In a preferred embodiment, the microarray comprises probes for measuring only RNAs that are listed in Table 1 (or subsets thereof).

In yet a further aspect, the invention also refers to a kit for the detection of TB in a human subject based on RNA obtained from a blood sample. Such a kit comprises a means for measuring the abundance of at least 4 RNAs that are chosen from the RNAs listed in Table 3. The means for measuring expression can be probes that allow for the detection of RNA in the sample or primers that allow for the amplification of RNA in the sample. Ways to devise probes and primers for such a kit are known to a person of skill in the art.

Further, the invention refers to the use of a kit as described above and herein for the detection of TB in a human subject based on RNA from a blood sample comprising means for measuring the abundance of at least 4 RNAs that are chosen from the RNAs listed in Table 1. Such a use may comprise the following steps: contacting at least one component of the kit with RNA from a blood sample from a human subject, measuring the abundance of at least 4 RNAs (or more as described above and herein) that are chosen from the RNAs listed in Table 1 using the means for measuring the abundance of at least 4 RNAs, and concluding, based on the measured abundance, whether the subject has TB.

In yet a further aspect, the invention also refers to a method for preparing an RNA expression profile that is indicative of the presence or absence of TB, comprising: isolating RNA from a whole blood sample, and determining the level or abundance of from 4 to about 3000 RNAs, including at least 4 RNAs selected from Table 1.

Preferably, the expression profile contains the level or abundance of 300 RNAs or less, of 200 RNAs or less, of 150 RNAs or less, or of 100 RNAs or less. Further, it is preferred that at least 10 RNAs, at least 30 RNAs, at least 100 RNAs are listed in Table 1.

In yet a further aspect, the invention also refers to a microarray, comprising a solid support and a set of oligonucleotide probes, the set containing from 4 to about 3,000 probes, and including at least 4 probes selected from Table 1. Preferably, the set contains 3000 probes or less (such as e.g. 200 probes, or less). At least 10 probes can be those listed in Table 1. At least 30 probes can be those listed in Table 1. In another embodiment, at least 100 probes are listed in Table 1.

Features of the invention that were described herein in combination with a method, a microarray, a kit, or a use also refer, if applicable, to all other aspects of the invention.

We chose primary molecular diagnosis for active tuberculosis (TB) since undetected patients with active TB are mainly responsible for the spread of the disease. More than 1/3 of the world's population is infected with TB and still more than 1.7 Million people die from the disease every year. About 7 to 10 Million people are expected to have active TB at any given time.

As active TB has a low prevalence but can be a deadly disease if not diagnosed in time, a test used for screening or diagnosis of active TB would have to achieve sensitivity and specificity greater 70%, preferably greater 80%, preferably greater 85%, preferably >90% 149 samples (53 of them with active TB) were only included into the study when passing all quality control checks. Following recent guidelines suggested by the MAQC consortium the preferred methodologies for data processing, feature selection and classifier development were established using one of the datasets provided by the MAQC consortium prior to application to the TB dataset. As a first step of test development the 149 samples were distributed into three sets (training set (TS), validation cohorts V1 and V2) (Figure 1). This initial setting allowed the development of a classifier within TS and two independent validations (in V1 resp. V2). Since classifier performance in subsequent validation cohorts might be strongly influenced by the characteristics of the patient population within TS, it was already suggested in MAQC-II to perform swap analysis of training and validation cohorts. In principle, swapping independent patient cohorts is just a special case of random permutation of samples. We therefore extended this approach from a single swap to 10.000 permutations (termed '10.000 trial simulation approach'; TSA) containing a TS and two independent validation cohorts (Fig. 1). Applied to TB, a mean AUC of between 0.86 and 0.90 (V1) resp. between 0. 86 and 0.90 (V2) was achieved by TSA (Fig. 1B). To elucidate dependency on methodology we varied SVM settings and compared SVM to LDA and PAM algorithms (n=8 x 10.000 classifier, Fig. 1C). While the results of SVM were further optimized using a linear kernel combined with t-Test instead of radial kernel and Wilcoxon-Test neither PAM nor LDA reached similarly high mean AUC. This was similarly true when reading out MCC (Fig. 4). As TSA clearly established the framework for classifier performance (range, median, 75% percentile), the next issues addressed were the dependency of classifier performance on feature size (n=5x10.000 classifier, Fig. 4), the sample distribution in TS (n=5x10.000 classifier) and the sample size in TS (n=49) using TSA (Fig 4). Unexpectedly, reducing feature size resulted in inferior classifier performance suggesting that due to the overall small sample size, more features are required to correctly classify in independent validation cohorts (Fig. 4). Taken together, TSA is well-suited to establish overall classifier performance that can be expected independent of the actual clinical situation with subsequent patient recruitment into TS and validation cohorts. Moreover, dependencies of classifier performance are easily uncovered.

We also determined the number of classifiers, reading a sufficiently high AUC (Fig. 5). About 7.8% of all classifiers reached an AUC of greater 0.95, 35% an AUC > 0.9, 86% an AUC > 0.8. Assessing the MCC showed similar results. To elucidate feature distribution all transcripts (n=48000) were evaluated for being part of at least one of 60.000 classifiers (Figs. 3). While a total of 41870 transcripts were part of at least one classifier in the TB dataset, only 68 transcripts were part of > 50% of all transcripts, 44 transcripts of >60%, 27 transcripts of >70%, 15 transcripts of >80%, and 6 transcripts of > 90% of classifiers. When assessing feature size and classifier performance (here AUC), an enormous variance became apparent in the TB dataset (Fig 2). There was a reduced variability of AUC when feature size was increased (see most right part of the figure versus most left part of the figure)

Together, these results support a robust gene expression profiling-based classifier as a test for primary diagnosis of active TB with a sensitivity and specificity of at least 80%, most likely >90%, most likely >95%.

The Invention is further described in the following Examples and Figures, which are, however, not to be construed as limiting the invention.

### Examples

Methods Summary: In this study a total of 149 gene expression samples (GEP) profiling were included. Following suggested standards by MAQC-II a procedure for data processing, feature selection and classifier optimization was developed using Bioconductor (R-packages) as the basis for all subsequent assessments. Using a dataset of peripheral blood derived GEP samples (n=149) a permutation approach (n = 10.000) to simulate a typical clinical pilot trial setting (trial simulation approach, TSA) consisting of three small but independent patient cohorts, here termed 'training set' (TS), validation set 1 (V1), and validation set 2 (V2) (Figure 1A). To assess patient distribution issues and classifier performance range and dependencies in this permutation approach diagnosis of active tuberculosis (TB) was chosen as a primary endpoint. Using AUC, MCC, sensitivity and specificity as readouts for the generated classifiers, 1) the range of performance of the classifiers, and 2) their dependency on a) classifier algorithms used, b) feature size, c) sample size within TS, and d) class distribution was assessed.

Description of gene expression profiling (GEP) dataset: A dataset established to detect patients with active tuberculosis was utilized. The dataset comprised all samples (n=149) from a recent study using whole blood derived GEP signatures to detect patients with active tuberculosis (http://www.nature.com/doifinder/10,1038/nature09247). In this study the Illumina platform utilizing Illumina Human HT-12 V3 BeadChip arrays was applied.

Protocol of decision making durin feature selection and model development.

Following recommendations made by MAQC-II the decision making process has been documented electronically. All statistical and bioinformatical analysis were performed using R software (www.r-project.org) version 2.10.1 and packages from the bioconductor project (htt://www.bioconductor.org/). Using one of the datasets (multiple myeloma) provided by the MAQC-II project (GSE24080) the preferred protocol for decision making during feature selection and model development was established. In principle, raw data (for Affymetrix: CEL-files) were downloaded from the GEO website and prepared for further analysis by R software.

Quality check procedure: Samples were subjected to an extended quality check prior use. First, a visual inspection of the distribution of raw expression values was performed using pairwise scatterplots of expression values from all arrays of a dataset. Overall median correlation within a combined dataset was required to be above 0.8. Next, the present call rate had to reach a threshold determined within the dataset as median present rate > 0.3. Third overall sample distribution was visually analyzed by density plots.

Normalization procedures: Illumina arrays were normalized according to approaches known in the art.

Batch-effect removal: Due to our overall strategy and to be able to better mimic expected clinical routine of subsequent data generation, which is naturally prone to batch-effects, we voted against batch-effect removal.

Feature selection approach: As the preferred feature selection approach we used a combination of fold changes and p-values based on t-tests (2-sided, unequal variance = Welch test, unpaired; "FC+P") between experimental groups in the training set. The p-values were adjusted for multiple testing using Benjamini-Hochberg correction. If not stated otherwise we used a FC ≥ 2 and p<0.05. To minimize overfitting issues the number of features was usually kept small. Overall, endpoints with a higher predictability showed a larger number of features that could be successfully used to obtain efficient classifiers, an observation that also became apparent during MAQC-II.

Classification algorithms: During setup we tested several classification algorithms (support vector machine (SVM), linear discrimination analysis (LDA), or prediction analysis for microarrays (PAM)). The different classifier were built and optimized based on the training set using a 10-fold cross-validation design repeated 10-times. The training set was divided 10 times into an internal training and an internal validation set in a ratio 9:1 (distribution to internal validation group see supplementary Table 1). In the internal training set the differentially expressed genes between positive and negative samples (cases and controls) were calculated using a t-test. Using the feature list extracted following the feature selection method, the different algorithms were trained on the internal training set and used to calculate the probability score for each case of the respective internal validation set. This approach was repeated 10 times according to the 10 dataset splitting of this 10 fold cross-validation. For each of the 10 cross-validation steps the area under the receiver operator curve (AUC) and the median Mathews Correlation Coefficient (MCC) were calculated for the internal validation set. The optimal classification algorithm was selected according to the maximum AUC and the maximum MCC reached in all algorithms tested. In our hands, SVM performed best and was therefore chosen for further analysis.

Validation of the optimized classifier: The optimized classifier was then applied to the validation cohorts. AUC and median MCC were used to measure the quality of the classifier. Sensitivity and specificity were calculated at the maximum Youden-index (sensitivity + specificity - 1). AUC and MCC values were calculated using prediction probabilities as implemented in the ROCR package. For description of specificity controls see the following paragraph.

Randomized permutation of training and validation sets: To assess robustness of classification, a permutation approach was applied, where each classification was repeated in 10.000 iterations (Trial Simulation Approach, TSA). In this re-sampling design, the dataset was randomly divided in one training set and two validation sets. If not stated otherwise, all divided sets comprise one third of the entire dataset. The classifier was built in the training set based on differentially expressed genes selected by statistical testing in the training set and then applied to the two independent validation sets.

### Discussion of Experiments with reference to the Figures

In total gene expression profiling samples derived from peripheral blood mononuclear cells were collected from public databases (GEO), quality controlled, and normalized for further analysis. A total of 149 samples passed all quality check filters.

To develop classifiers to diagnose active TB the procedure shown in Fig. 1 was performed initially. First, 149 samples were divided into three independent datasets. One of the three datasets was set to become the training set (TS), one the first validation cohort (V1) and the third dataset to become the second validation cohort (V2). Within TS, the classifier was generated using a defined approach for feature selection, a defined classification algorithm and a 10x cross validation (internal validation). The classifier build in TS was then validated in the independent datasets V1 and V2. To better understand the role of feature size classification algorithm, ratio of cases and controls as well as size of training set, these variables were varied (see figures below). The setting described so far, could reflect a clinical situation where TS would have been drawn first, followed by V1 and V2. However, a classifier identified in such a typical clinical setting might not be generalizable. In fact, it might be biased and might underperform. To address the influence of patient entry into the independent cohorts, 10.000 clinical trial settings (Trial Simulation Approach, TSA) were tested and for each setting a classifier vor V1 respectively V2 determined. In such a scenario, it is possible to determine the average performance as well as the 25, 75, 90, 95 quantiles of test performances to be expected irrespective of patient entry.
Fig. 4 shows the graphical representation of the AUC of each of the 10.000 classifiers in V1 (grey) respectively V2 (red). Mean (line in grey box), 25/75 percentiles (boxes) and 95 percentiles (lines) as well as outliers (dots) are shown. Number of Features was varied by applying different Feature selection criteria. The data clearly demonstrate that there is no significant difference between V1 and V2 concerning the AUC results of the 10.000 classifiers. The data also present that already rather high AUC values can be observed using only a rather small number of samples. As further statistical readouts of classifier performance MCC, sensitivity and specificity have been performed as well. These data are available upon request. In Figur 4, the influence of feature size in classifier performance is shown. The number of features was altered based on a filter combining different levels of significance (p-value) and fold change for each feature (transcript on the array) between the groups active TB and non-active TB. More important, for both validation cohorts, the classifier performance was better with higher numbers of features (FC > 4 = more features)

The small dataset already results in a sufficiently high AUC in the majority of classifiers developed (Fig. 5). About 7.8% of all classifiers reached an AUC of greater 0.95, 35% an AUC > 0.9, 86% an AUC > 0.8. Assessing the MCC showed similar results.

To elucidate feature distribution all transcripts (n=**48000**) were evaluated for being part of at least one of 60.000 classifiers (Fig. 3). While a total of 41870 transcripts were part of at least one classifier in the TB dataset, only 68 transcripts were part of > 50% of all transcripts, 44 transcripts of >60%, 27 transcripts of >70%, 15 transcripts of >80%, and 6 transcripts of > 90% of classifiers. When assessing feature size and classifier performance (here AUC), an enormous variance became apparent in the TB dataset (Fig 2). There was a reduced variability of AUC when feature size was increased (see most right part of the figure versus most left part of the figure)

These data indicate that there is a small set of transcripts that are always part of a classifier irrespective of the distribution of patients into test and validation sets. These few transcripts are the prime candidates for building the test for the primary diagnosis of active TB.

Together, these results support a robust gene expression profiling-based classifier as a test for primary diagnosis of active TB with a sensitivity and specificity of greater 80%, preferably 90%, preferably >95%.

**Table 1**

| SEQ ID | Ilummina | Annoy | mean_TB | mean_controls | FC |
|---|---|---|---|---|---|
| 1 | ILMN-2114568 | NM_052942 | 3486,71811 | 735,3565548 | 4,741534 |
| 2 | ILMN_2261600 | NM_001017986 | 2268,81101 | 362,6405648 | 6,25636299 |
| 3 | ILMN_2391051 | NM_001004340 | 723,621752 | 120,2649739 | 6,01689526 |
| 4 | ILMN_2176063 | NM_000566 | 1412,74636 | 209,2325696 | 6,75203849 |
| 5 | ILMN_1851599 | Hs30245 | 289,618119 | 46,06176818 | 6,28760316 |
| 6 | ILMN_17O1114 | NM_002053 | 1351,98133 | 355,8667438 | 3,79912244 |
| 7 | ILMN_1690241 | NM_138456 | 185,177747 | 29,23425054 | 6,33427378 |
| 8 | ILMN_1670305 | NM_001032295 | 262,69742 | 41,102674 | 6,3912489 |
| 9 | ILMN_1809467 | NM_006634 | 1142,7623 | 370,7487699 | 3,08230909 |
| 10 | ILMN_2394210 | NM_138718 | 51,2289871 | 13,60973733 | 3,76414223 |
| 11 | ILMN_1654389 | XM_001128342 | 172,411623 | 24,7307261 | 6,97155525 |
| 12 | ILMN_1727271 | NM_173701 | 1478,7956 | 473,7437882 | 3,12150922 |
| 13 | ILMN_2388547 | NM_033255 | 3461,43505 | 1176,03598 | 2,9433071 |
| 14 | ILMN_2148785 | NM_002053 | 1735,9282 | 462,6477925 | 3,75215927 |
| 15 | ILMN_2408987 | NM_001003802 | 31,4874504 | 6,23923582 | 5,04668381 |
| 16 | ILMN_1701914 | NM_014143 | 49,3740188 | 10,98372163 | 4,49519939 |
| 17 | ILMN_1805750 | NM_021034 | 8685,31302 | 2655,368157 | 3,27085079 |
| 18 | ILMN_1755843 | NM_052961 | 299,975496 | 100,8666628 | 2,97398057 |
| 19 | ILMN_2302757 | NM_003890 | 98,6807465 | 273,3345674 | 0,36102549 |
| 20 | ILMN_1664330 | NM_001712 | 171,034067 | 63,47031271 | 2,6947097 |
| 21 | ILMN_1701621 | NM_005138 | 1061,59269 | 399,938371 | 2,65439069 |
| 22 | ILMN_1681301 | NM_004833 | 1677,09758 | 411,6203677 | 4,07437948 |
| 23 | ILMN_2337655 | NM_004184 | 2746,41171 | 937,1662 | 2,93054926 |
| 24 | ILMN_1713058 | NM_024430 | 561,60975 | 201,2801859 | 2,79018895 |
| 25 | ILMN_1675756 | NM_170736 | 684,568474 | 249,7157788 | 2,74139054 |
| 26 | ILMN_1662731 | NM_031365 | 3,37436283 | 11,28487182 | 0,2990165 |
| 27 | ILMN_1747744 | NM_005779 | 227,231797 | 83,46996946 | 2,72231796 |
| 28 | ILMN_1690105 | NM_007315 | 1751,67712 | 700,5415441 | 2,50046144 |
| 29 | ILMN_1776157 | NM_080415 | 270,164281 | 53,81394312 | 5,02033981 |
| 30 | ILMN_1707979 | NM_001007232 | 110,670937 | 20,43749637 | 5,41509269 |
| 31 | ILMN_1860051 | Hs37991 | 70,3463081 | 13,95994732 | 5,03915283 |
| 32 | ILMN_2374865 | NM_001040619 | 61,0771971 | 16,28053609 | 3,75154705 |
| 33 | ILMN_1771385 | NM_052941 | 769,176867 | 289,99671 | 2,65236411 |
| 34 | ILMN_1683792 | NM_015907 | 759,34755 | 282,6253774 | 2,68676351 |
| 35 | ILMN_1740572 | NM_000355 | 27,9417966 | 9,658522327 | 2,89296806 |
| 36 | ILMN_2309180 | NM_003078 | 214,337353 | 79,71044989 | 2,68894923 |
| 37 | ILMN_1776939 | NM_152866 | 17,518465 | 42,45100338 | 0,41267493 |
| 38 | ILMN_1780831 | NM_003044 | 67,3510228 | 26,64039015 | 2,52815452 |
| 39 | ILMN_1733998 | NM_005771 | 2017,83053 | 833,5127946 | 2,42087529 |
| 40 | ILMN_1701455 | NM_018438 | 303,421487 | 125,753207 | 2,41283299 |
| 41 | ILMN_2390946 | NM_145350 | 14,4448906 | 3,682560611 | 3,92251264 |
| 42 | ILMN_2393296 | NM_203391 | 591,190259 | 233,2520281 | 2,53455571 |
| 43 | ILMN_1887868 | Hs45973 | 34,2704059 | 11,46663206 | 2,98870721 |
| 44 | ILMN_1700671 | NM_016135 | 41,300438 | 10,89540535 | 3,79062886 |
| 45 | ILMN_1716815 | NM_001024912 | 690,577898 | 276,6717061 | 2,49601923 |
| 46 | ILMN_1755203 | NM_001081 | 2,50761918 | 8,231640867 | 0,30463175 |
| 47 | ILMN_1810289 | NM_133337 | 266,499908 | 110,3188039 | 2,41572514 |
| 48 | ILMN_2173975 | NM_022147 | 189,235825 | 76,43536774 | 2,47576261 |
| 49 | ILMN_1669497 | NM_017784 | 25,8755199 | 63,05484372 | 0,4103653 |
| 50 | ILMN_1782487 | NR_003133 | 279,350117 | 63,80442817 | 4,3782246 |
| 51 | ILMN_1759075 | NM_012452 | 13,2134347 | 32,36935457 | 0,40820816 |
| 52 | ILMN_1725471 | NM_000167 | 624,266833 | 257,5272947 | 2,42408027 |
| 53 | ILMN_1703881 | XM_928247 | 1,2761118 | 7,550238 | 0,1690161 |
| 54 | ILMN_2396903 | NM_170737 | 98,0943602 | 34,428298 | 2,84923641 |
| 55 | ILMN_1859584 | Hs06876 | 106,160608 | 31,16459302 | 3,40644935 |
| 56 | ILMN_1691071 | NM_032738 | 123,241223 | 280,4614063 | 0,43942311 |
| 57 | ILMN_1688631 | NM_145343 | 17,2833771 | 4,071637509 | 4,24482216 |
| 58 | ILMN_1756953 | NM_198460 | 169,719709 | 17,93658009 | 9,4622112 |
| 59 | ILMN_2342835 | NM_014879 | 176,742887 | 37,29518737 | 4,73902665 |
| 60 | ILMN_1785732 | NM_007115 | 2344,01621 | 819,2418796 | 2,86120164 |
| 61 | ILMN_2092850 | NM_006665 | 155,920896 | 65,35690269 | 2,38568367 |
| 62 | ILMN_2371724 | NM_001024912 | 453,941409 | 187,0596768 | 2,42671974 |
| 63 | ILMN_2126706 | NM_005573 | 245,42658 | 105,657003 | 2,32286146 |
| 64 | ILMN_1799848 | NM_144590 | 588,732245 | 45,02711423 | 13,0750606 |
| 65 | ILMN_1722981 | NM_003268 | 535,908444 | 232,1603332 | 2,30835491 |
| 66 | ILMN_1748529 | NM_005059 | 2,59477273 | 8,544664732 | 0,30367168 |
| 67 | ILMN_2258409 | NM_014879 | 26,2525691 | 3,742377955 | 7,01494327 |
| 68 | ILMN_2384181 | NM_005771 | 563,496947 | 213,4969552 | 2,6393676 |
| 69 | ILMN_1693630 | NM_004913 | 466,771822 | 199,2461651 | 2,34268911 |
| 70 | ILMN_1745242 | NM_021105 | 133,339937 | 54,24976998 | 2,45788944 |
| 71 | ILMN_1683250 | XM_933693 | 252,603212 | 101,5200338 | 2,48821048 |
| 72 | ILMN_1765332 | NM_012456 | 737,462122 | 295,30265 | 2,49730953 |
| 73 | ILMN_1652277 | NM_003004 | 18,4746549 | 6,797859809 | 2,71771637 |
| 74 | ILMN_1691364 | NM_139266 | 6400,62633 | 2894,588143 | 2,21123905 |
| 75 | ILMN_1701789 | NM_001031683 | 3718,20684 | 1603,565601 | 2,31871202 |
| 76 | ILMN_1769520 | NM_004223 | 5047,62946 | 2298,319542 | 2,19622614 |
| 77 | ILMN_1703108 | NM_004223 | 662,635072 | 301,7860333 | 2,19571153 |
| 78 | ILMN_1671703 | NM_001613 | 391,270721 | 178,1269105 | 2,19658399 |
| 79 | ILMN_2049184 | NM_004944 | 9,87427097 | 22,02558343 | 0,44830917 |
| 80 | ILMN_1710740 | NM_000063 | 25,3080744 | 11,54420737 | 2,19227475 |
| 81 | ILMN_1797522 | NM_004090 | 1274,9514 | 564,4740968 | 2,25865351 |
| 82 | ILMN_2204876 | NM_017791 | 206,499032 | 84,60822548 | 2,4406496 |
| 83 | ILMN_1661673 | NR_003141 | 2,13029449 | 6,07280189 | 0,35079269 |
| 84 | ILMN_2167426 | NM_020437 | 127,970163 | 58,8186329 | 2,17567388 |
| 85 | ILMN_1762531 | NM_002010 | 2,8027006 | 9,552363181 | 0,2934039 |
| 86 | ILMN_1762713 | NM_174918 | 1048,10473 | 363,4616641 | 2,88367339 |
| 87 | ILMN_2402172 | NM_080416 | 26,1965252 | 3,347717339 | 7,8251903 |
| 88 | ILMN_1659227 | NM_001783 | 36,6190502 | 77,92261925 | 0,46994121 |
| 89 | ILMN_1779558 | NM_000820 | 56,269164 | 25,35818972 | 2,21897401 |
| 90 | ILMN_1745374 | NM_005533 | 986,39593 | 457,0075849 | 2,15837978 |
| 91 | ILMN_1664047 | NM_000721 | 68,8580421 | 20,29464428 | 3,39291693 |
| 92 | ILMN_1672575 | NM_138718 | 31,9086266 | 14,75932763 | 2,16192956 |
| 93 | ILMN_1775235 | NM_001025108 | 42,9803545 | 90,85523 | 0,47306418 |
| 94 | ILMN_1809817 | NM_152750 | -0,53979704 | 4,457647682 | -0,1210946 |
| 95 | ILMN_1726545 | NM_181879 | 101,693593 | 43,02251601 | 2,36372957 |
| 96 | ILMN_1672022 | NM_004438 | 17,4078391 | 37,2137125 | 0,46778023 |
| 97 | ILMN_1796138 | NM_001721 | 32,0333199 | 12,74734711 | 2,51294011 |
| 98 | ILMN_2121568 | NM_198460 | 30,4470907 | 4,967416001 | 6,12936197 |
| 99 | ILMN_1722158 | NM_004347 | 333,418862 | 107,5355062 | 3,10054673 |
| 100 | ILMN_1653648 | NM_030784 | 7,48444671 | 1,592310044 | 4,70037022 |
| 101 | ILMN_1681846 | NM_152606 | 13,9755259 | 29,56961039 | 0,47263139 |
| 102 | ILMN_1837219 | Hs63426 | 1,88159367 | 7,603388019 | 0,2474678 |
| 103 | ILMN_1753758 | NM_145659 | 11,3212904 | 0,738651159 | 15,3269784 |
| 104 | ILMN_1667114 | NR_003662 | 4,04963548 | 8,989281987 | 0,45049599 |
| 105 | ILMN_1687960 | NM_016733 | 76,6374322 | 33,37796159 | 2,29604891 |
| 106 | ILMN_1660624 | NM_001031801 | 110,594426 | 50,20865431 | 2,20269648 |
| 107 | ILMN_1710734 | NM_002104 | 440,719063 | 920,8234226 | 0,47861409 |
| 108 | ILMN_1712719 | NM_003980 | 20,2604735 | 47,24787296 | 0,42881239 |
| 109 | ILMN_2362232 | NM_018555 | 4,12074509 | 12,32671754 | 0,33429379 |
| 110 | ILMN_2227195 | NM_033124 | 14,8816762 | 31,75706869 | 0,46860988 |
| 111 | ILMN_1725417 | NM_006159 | 169,444309 | 355,8244086 | 0,47620204 |
| 112 | ILMN_1798270 | NM_020179 | 320,333495 | 150,7672518 | 2,12468882 |
| 113 | ILMN_2144426 | NM_003516 | 2997,18502 | 1308,353058 | 2,29080752 |
| 114 | ILMN_1910908 | Hs.72010 | 53,812966 | 21,75360176 | 2,47374971 |
| 115 | ILMN_1834211 | Hs31296 | 12,973675 | 3,984481081 | 3,25605134 |
| 116 | ILMN_1778681 | NM_024007 | 24,3377725 | 52,16448053 | 0,46655832 |
| 117 | ILMN_1731503 | NM_006770 | 36,9691203 | 13,6192067 | 2,71448412 |
| 118 | ILMN_1777792 | XM_944064 | 3,99123983 | 10,8685622 | 0,36722795 |
| 119 | ILMN_1694548 | NM_005139 | 104,53479 | 38,4839643 | 2,71632074 |
| 120 | ILMN_2143314 | NM_003121 | 20,7025491 | 44,10713353 | 0,46936963 |
| 121 | ILMN_1779252 | NM_006074 | 1293,59439 | 620,511529 | 2,08472258 |
| 122 | ILMN_1799467 | NM_152703 | 751,164109 | 356,6040933 | 2,10643715 |
| 123 | ILMN_1734276 | NM_199169 | 7,91780972 | 16,58333185 | 0,47745591 |
| 124 | ILMN_2159272 | NM_025239 | 4,99074127 | -6,630165266 | -0,75273256 |
| 125 | ILMN_1796409 | NM_000491 | 110,63042 | 14,7162795 | 7,51755362 |
| 126 | ILMN_1659770 | NM_170736 | 26,7933665 | 9,293667539 | 2,88297019 |
| 127 | ILMN_1731224 | NM_031458 | 1901,26614 | 921,5357129 | 2,06314971 |
| 128 | ILMN_1808979 | NM_080387 | 99,0661693 | 37,87220608 | 2,61580139 |
| 129 | ILMN_1735792 | NM_001005498 | 49,5635214 | 23,05900896 | 2,14942114 |
| 130 | ILMN_1708934 | NM_001124 | 3520,89983 | 1616,374176 | 2,17827028 |
| 131 | ILMN_1772466 | NM_005490 | 5,40880321 | 11,31577176 | 0,47798801 |
| 132 | ILMN_1659047 | NM_003516 | 1533,32927 | 717,6836849 | 2,13649732 |
| 133 | ILMN_2337928 | NM_032966 | 80,6921029 | 165,2142359 | 0,4884089 |
| 134 | ILMN_1657868 | NM_012240 | 8,46669902 | 17,34565204 | 0,4881165 |
| 135 | ILMN_2132599 | NM_144590 | 693,642299 | 52,32126641 | 13,2573683 |
| 136 | ILMN_1774287 | NM_001710 | 7,98802936 | 1,043680862 | 7,65370876 |
| 137 | ILMN_2370976 | NM_013451 | 45,0366991 | 21,25398513 | 2,11897669 |
| 138 | ILMN_1751079 | NM_000593 | 5528,95506 | 2707,965258 | 2,04173781 |
| 139 | ILMN_1674620 | NM_001099400 | 5,09169357 | 12,06271064 | 0,42210194 |
| 140 | ILMN_1699599 | NM_052939 | 20,6646539 | 42,75662439 | 0,48330882 |
| 141 | ILMN_2337386 | NM_031200 | -1,70220011 | 6,340900359 | -0,2684477 |
| 142 | ILMN_1812759 | NM_001024071 | 208,292872 | 101,2324344 | 2,05757052 |
| 143 | ILMN_1676780 | NM_018556 | 9,4319171 | 21,67619102 | 0,43512797 |
| 144 | ILMN_1652287 | NM_005450 | 4,20722069 | 9,850311761 | 0,42711549 |
| 145 | ILMN_1903064 | Hs61105 | 48,7733252 | 23,72495617 | 2,05578147 |
| 146 | ILMN_1734878 | NM_021601 | 389,629903 | 782,6926548 | 0,49780703 |
| 147 | ILMN_1887011 | Hs11718 | 8,78221169 | 20,9798904 | 0,41860141 |
| 148 | ILMN_1668277 | NM_001715 | 31,8957465 | 65,41084194 | 0,48762171 |
| 149 | ILMN_1810420 | NM_003494 | 1417,39558 | 684,0669828 | 2,07201285 |
| 150 | ILMN_1750294 | NM_003817 | 28,1511574 | 56,06408704 | 0,5021246 |
| 151 | ILMN_1759772 | NM_198075 | 4,71164402 | 11,11973372 | 0,42371914 |
| 152 | ILMN_2062620 | NM_004808 | 14,3666837 | 28,82665067 | 0,498382 |
| 153 | ILMN_1778059 | NM_033306 | 123,602054 | 59,35378129 | 2,08246301 |
| 154 | ILMN_2279844 | NM_005356 | 12,3119898 | 24,26360355 | 0,50742627 |
| 155 | ILMN_1673503 | NM_001005747 | 5,29894702 | 11,37564702 | 0,465815 |
| 156 | ILMN_1732296 | NM_002167 | 81,5329234 | 160,9985376 | 0,50642027 |
| 157 | ILMN_2052717 | NM_017577 | 11,8473405 | 24,19690768 | 0,48962209 |
| 158 | ILMN_1811364 | XM_938742 | 7,18716689 | 14,9152896 | 0,48186573 |
| 159 | ILMN_1677693 | NM_006018 | 202,212168 | 99,2322272 | 2,0377671 |
| 160 | ILMN_1690907 | NM_031409 | 89,8332813 | 177,8786297 | 0,50502571 |
| 161 | ILMN_1815673 | NM_015881 | 0,91491099 | 7,641781475 | 0,11972483 |
| 162 | ILMN_1807423 | NM_006547 | 104,241476 | 49,70447872 | 2,09722501 |
| 163 | ILMN_1707077 | NM_002959 | 248,829025 | 124,8174614 | 1,99354339 |
| 164 | ILMN_1691731 | NM_017554 | 783,457052 | 393,3101903 | 1,99195717 |
| 165 | ILMN_1694966 | NM_080914 | 108,492881 | 49,8674711 | 2,17562429 |
| 166 | ILMN_2168217 | NM_004951 | 113,969909 | 224,4873952 | 0,50768957 |
| 167 | ILMN_1741350 | NM_024491 | 3,28333767 | 8,647444887 | 0,37968876 |
| 168 | ILMN_1678494 | NM_182755 | 200,774907 | 96,34396237 | 2,08393865 |
| 169 | ILMN_1812091 | NM_017565 | 7,40834057 | 0,128523056 | 57,6421132 |
| 170 | ILMN_2306540 | NM_001001567 | 16,4186526 | 32,19625811 | 0,5099553 |
| 171 | ILMN_1798706 | NM_004951 | 300,341378 | 587,2299581 | 0,51145445 |
| 172 | ILMN_2244009 | NM_030915 | 15,6279178 | 31,12367759 | 0,50212311 |
| 173 | ILMN_1797428 | NM_052939 | 33,4866905 | 67,52726398 | 0,49589882 |
| 174 | ILMN_2367671 | NM_016733 | 79,7178046 | 38,24625834 | 2,0843295 |
| 175 | ILMN_2401987 | NM_007337 | 7,89865165 | 15,35728278 | 0,51432612 |
| 176 | ILMN_1690939 | NM_001953 | 133,713816 | 67,84330817 | 1,97092122 |
| 177 | ILMN_1785345 | NM_020370 | 53,0480909 | 12,95422085 | 4,09504296 |
| 178 | ILMN_2066849 | NM_001010919 | 194,633206 | 88,07278871 | 2,20991306 |
| 179 | ILMN_1657760 | NM_016524 | 2,76827094 | 9,063093959 | 0,30544436 |
| 180 | ILMN_1773650 | NM_001099660 | 147,764805 | 295,1965981 | 0,50056405 |
| 181 | ILMN_1656910 | NM_058166 | 47,6688796 | 21,6450953 | 2,20229474 |
| 182 | ILMN_1777325 | NM_007315 | 3486,2063 | 1738,591683 | 2,00518979 |
| 183 | ILMN_1656849 | NM_052961 | 19,4481733 | 6,032030419 | 3,2241504 |
| 184 | ILMN_1806149 | NM_206967 | 4,73970169 | 9,762427122 | 0,48550444 |
| 185 | ILMN_2230862 | NM_004130 | 974,529219 | 488,2044409 | 1,99614984 |
| 186 | ILMN_1710698 | XM_937794 | 21,0210096 | 10,30588325 | 2,03970965 |
| 187 | ILMN_1672097 | NM_006889 | 5,29181414 | 10,96253957 | 0,48271791 |
| 188 | ILMN_1768973 | NM_003517 | 1607,98329 | 724,2788559 | 2,22011629 |
| 189 | ILMN_1798971 | NM_003468 | 9,43512377 | 3,880958903 | 2,4311321 |
| 190 | ILMN_1782704 | NM_001770 | 152,173719 | 296,6521554 | 0,51297021 |
| 191 | ILMN_1681601 | NM_033050 | 40,8036281 | 18,41058375 | 2,21631365 |
| 192 | ILMN_1801307 | NM_003810 | 2517,05096 | 1286,824044 | 1,95601797 |
| 193 | ILMN_1669321 | NM_139355 | 11,4154943 | 22,39022902 | 0,50984268 |
| 194 | ILMN_1732291 | XM_931236 | 7,8531964 | 15,14166745 | 0,51864806 |
| 195 | ILMN_1750497 | NM_177551 | 149,021331 | 73,69175785 | 2,02222522 |
| 196 | ILMN_1787845 | XM_942700 | 3,84980027 | 8,869502166 | 0,43404919 |
| 197 | ILMN_1801313 | NM_005067 | 559,701269 | 263,0821639 | 2,12747706 |
| 198 | ILMN_2371079 | NM_000869 | 4,78747487 | 10,44398552 | 0,4583954 |
| 199 | ILMN_1661809 | NM_024081 | 115,245036 | 58,73623957 | 1,96207719 |
| 200 | ILMN_1724789 | NM_000611 | 27,9341096 | 13,78676484 | 2,02615407 |
| 201 | ILMN_2229261 | NR_002825 | 58,5022663 | 29,88484239 | 1,95758992 |
| 202 | ILMN_2346836 | NM_001080439 | 3,37389011 | 9,304226528 | 0,36261909 |
| 203 | ILMN_2357419 | NM_021250 | 332,608155 | 160,4780867 | 2,07260793 |
| 204 | ILMN_2216815 | NM_003980 | 18,6720682 | 40,6872551 | 0,45891688 |
| 205 | ILMN_1702120 | NM_199074 | 55,6030792 | 27,05102984 | 2,05548844 |
| 206 | ILMN_1792455 | NM_015444 | 933,937544 | 402,8261656 | 2,31846296 |
| 207 | ILMN_1667022 | NM_015148 | 76,2390363 | 143,5500988 | 0,53109707 |
| 208 | ILMN_2342638 | NM_080914 | 266,233504 | 118,2585629 | 2,2512831 |
| 209 | ILMN_1793578 | NM_003408 | 10,8851533 | 21,39077939 | 0,50887128 |
| 210 | ILMN_1695435 | XM_928387 | 106,76024 | 49,10914022 | 2,17393829 |
| 211 | ILMN_2398159 | NM_013253 | 2,62966369 | 11,52142245 | 0,22824123 |
| 212 | ILMN_1786281 | NM_001039643 | 2,63745939 | 6,783977206 | 0,38877775 |
| 213 | ILMN_2347798 | NM_022872 | 1345,6366 | 611,5649074 | 2,20031689 |
| 214 | ILMN_1664063 | NM_173544 | 36,7515738 | 70,34032789 | 0,52248226 |
| 215 | ILMN_2395214 | NM_198900 | 13,3747742 | 25,42768473 | 0,52599261 |
| 216 | ILMN_1754858 | NM_015148 | 55,4913374 | 103,2977722 | 0,53719781 |
| 217 | ILMN_1774733 | NM_003745 | 94,8660013 | 27,37419225 | 3,46552696 |
| 218 | ILMN_1676003 | NM_006228 | 14,5743711 | 27,89358746 | 0,52249899 |
| 219 | ILMN_2369221 | NM_172174 | 26,8317301 | 12,2480685 | 2,19069073 |
| 220 | ILMN_2209614 | NM_001014440 | 8,07629909 | 3,551072525 | 2,27432671 |
| 221 | ILMN_1822671 | Hs67460 | 27,694373 | 61,653435 | 0,44919432 |
| 222 | ILMN_2098013 | NM_000078 | 80,8270983 | 40,30143559 | 2,00556375 |
| 223 | ILMN_2326273 | NM_004000 | 23,6052984 | 45,51337462 | 0,51864531 |
| 224 | ILMN_1772697 | NM_001039571 | 38,4689847 | 8,787357144 | 4,37776502 |
| 225 | ILMN_1812433 | NM_005143 | 46,192577 | 10,84292152 | 4,26015967 |
| 226 | ILMN_2325337 | NM_145637 | 109,358923 | 49,26357686 | 2,21987378 |
| 227 | ILMN_1785902 | NM_172369 | 24,9981865 | 2,248841197 | 11,1160301 |
| 228 | ILMN_1754076 | NM_018398 | 26,3564843 | 50,06322462 | 0,52646398 |
| 229 | ILMN_1784749 | NM_000820 | 51,9792396 | 26,64802568 | 1,95058502 |
| 230 | ILMN_1708728 | NM_177925 | 566,811261 | 274,9804258 | 2,06127858 |
| 231 | ILMN_1845037 | Hs89254 | 221,69789 | 112,8579726 | 1,96439724 |
| 232 | ILMN_1721127 | NM_003530 | 83,4013641 | 40,10038885 | 2,07981435 |
| 233 | ILMN_2413615 | NM_001015002 | 5,89773949 | 11,37640303 | 0,51841865 |
| 234 | ILMN_2325338 | NM_145637 | 124,315156 | 64,42116258 | 1,92972544 |
| 235 | ILMN_1767377 | NM_207331 | 789,420428 | 358,8806767 | 2,19967382 |
| 236 | ILMN_1767260 | NM_144978 | 2,77346174 | 7,00201183 | 0,39609498 |
| 237 | ILMN_1742618 | NM_199139 | 1410,07214 | 723,7187409 | 1,94837036 |
| 238 | ILMN_2066151 | NM_000459 | 2,4102212 | 7,192279046 | 0,3351123 |
| 239 | ILMN_1726591 | NM_052889 | 390,615147 | 184,8085251 | 2,11362082 |
| 240 | ILMN_1770800 | NM_153703 | 5,96084022 | 13,9578254 | 0,42706081 |
| 241 | ILMN_2137084 | NM_173083 | 2,64106238 | 8,396196047 | 0,31455463 |
| 242 | ILMN_1745112 | NM_001035254 | 37,4394874 | 70,01580892 | 0,53472906 |
| 243 | ILMN_1692398 | NM_003632 | 6,63761813 | 12,99830611 | 0,51065255 |
| 244 | ILMN_1691717 | NM_001005498 | 1138,58976 | 594,8499538 | 1,91407893 |
| 245 | ILMN_1791329 | NM_030764 | 86,1089527 | 161,6268674 | 0,53276385 |
| 246 | ILMN_1775542 | NM_005449 | 829,113189 | 1561,546712 | 0,53095638 |
| 247 | ILMN_1651498 | NM_006705 | 51,1723429 | 20,19997295 | 2,53328769 |
| 248 | ILMN_1665682 | NM_002189 | 11,8964374 | 4,084263222 | 2,91274993 |
| 249 | ILMN_1724315 | NM_014916 | 22,3190929 | 9,061459158 | 2,46307934 |
| 250 | ILMN_1751576 | NM_000459 | 1,89050468 | 5,994427083 | 0,31537704 |
| 251 | ILMN_1697729 | NM_002758 | 1,38157734 | 6,106929502 | 0,22623109 |
| 252 | ILMN_1780292 | NM_002441 | 3,73431012 | 7,615556683 | 0,49035288 |
| 253 | ILMN_2058468 | NM_021813 | 59,9212733 | 116,0822042 | 0,51619689 |
| 254 | ILMN_2194561 | NM_001029884 | -0,03824889 | 5,665792723 | -0,00675085 |
| 255 | ILMN_1680757 | NM_001013653 | 10,3832983 | 19,88999601 | 0,52203622 |
| 256 | ILMN_2371458 | NM_001047841 | 7,59229423 | 14,44488445 | 0,52560436 |
| 257 | ILMN_2402640 | NM_004948 | 11,6771341 | 20,71767461 | 0,5636315 |
| 258 | ILMN_1765954 | NM_000197 | 2,12199246 | 6,361213772 | 0,33358295 |
| 259 | ILMN_1729799 | XM_932186 | 2,81273631 | 5,891800139 | 0,47739846 |
| 260 | ILMN_1661940 | NM_015215 | 9,00860694 | 17,16475454 | 0,52483168 |
| 261 | ILMN_1777261 | NM_001040020 | 16,3108483 | 31,1240941 | 0,52405857 |
| 262 | ILMN_1756705 | NM_022092 | 9,8516365 | 18,61951062 | 0,52910287 |
| 263 | ILMN_1718977 | NM_015675 | 192,098966 | 99,38252022 | 1,93292509 |
| 264 | ILMN_1872531 | Hs43636 | 2,30898364 | 5,290678115 | 0,43642489 |
| 265 | ILMN_1666221 | NM_001010925 | 5,51387939 | 10,22289896 | 0,53936554 |
| 266 | ILMN_1780546 | NM_020530 | 122,064578 | 54,23736968 | 2,25056228 |
| 267 | ILMN_1910120 | NR_003370 | 24,7014197 | 46,0161042 | 0,53679946 |
| 268 | ILMN_1766896 | NM_001039617 | 48,4533378 | 14,51931893 | 3,33716327 |
| 269 | ILMN_2335813 | NM_001024070 | 50,1997798 | 19,81290125 | 2,53369152 |
| 270 | ILMN_1671777 | NM_004114 | 8,07242524 | 0,681094159 | 11,8521428 |
| 271 | ILMN_1658016 | NM_178457 | 22,3885379 | 42,18221419 | 0,53075777 |
| 272 | ILMN_1728073 | NM_020946 | 23,7659846 | 12,51901178 | 1,89839143 |
| 273 | ILMN_1791890 | NM_006108 | 5,88921948 | 11,11825357 | 0,52968926 |
| 274 | ILMN_1700147 | NM_013378 | 93,3606477 | 176,3570318 | 0,52938432 |
| 275 | ILMN_2325506 | NM_017843 | 87,4554485 | 162,1942132 | 0,53920203 |
| 276 | ILMN_1721888 | NM_000419 | 202,661052 | 92,31057723 | 2,19542612 |
| 277 | ILMN_1686454 | NM_052864 | 60,5912617 | 20,89464174 | 2,89984688 |
| 278 | ILMN_2240221 | NM_032943 | 12,8838784 | 24,21342574 | 0,53209647 |
| 279 | ILMN_1657977 | NM_012228 | 1220,74331 | 647,9690495 | 1,88395311 |
| 280 | ILMN_1792075 | NM_001771 | 15,9083379 | 29,74846825 | 0,53476158 |
| 281 | ILMN_1664236 | NM_021777 | 16,9370246 | 31,9270379 | 0,53049157 |
| 282 | ILMN_2156250 | NM_003955 | 9,56781548 | 1,98148845 | 4,82860018 |
| 283 | ILMN_1661646 | NM_001083907 | 191,137283 | 354,5867194 | 0,53904242 |
| 284 | ILMN_2053527 | NM_031458 | 208,961488 | 98,6554847 | 2,11809296 |
| 285 | ILMN_2090004 | NM_080429 | 125,771703 | 61,18614319 | 2,0555586 |
| 286 | ILMN_2048607 | NM_152326 | 482,57755 | 249,3075951 | 1,93567127 |
| 287 | ILMN_1681310 | XM_001127310 | 17,6955982 | 9,5122105 | 1,86030347 |
| 288 | ILMN_1781077 | NM_153223 | 7,53604583 | 14,47164674 | 0,52074556 |
| 289 | ILMN_2355953 | NM_001081438 | 53,1328862 | 28,35731477 | 1,87369244 |
| 290 | ILMN_1711514 | NM_004086 | 18,9267996 | 36,33810247 | 0,52085272 |
| 291 | ILMN_1688299 | NM_013441 | 11,9666247 | 21,60587742 | 0,5538597 |
| 292 | ILMN_1740606 | NM_145307 | 3,22760494 | 8,152756142 | 0,39589126 |
| 293 | ILMN_1698725 | NM_174938 | 266,868807 | 141,2857506 | 1,88885861 |
| 294 | ILMN_1720511 | NM_020873 | 7,72159744 | 2,887596002 | 2,6740574 |
| 295 | ILMN_1699809 | NM_032330 | 10,8699725 | 5,244263215 | 2,07273587 |
| 296 | ILMN_1758400 | NM_133445 | 2,34194754 | -2,932477774 | -0,79862414 |
| 297 | ILMN_2261627 | NM_005214 | 8,29913866 | 15,51222671 | 0,53500628 |
| 298 | ILMN_1656560 | NM_015393 | 11,3401009 | 20,74740324 | 0,54657929 |
| 299 | ILMN_1792726 | NM_006862 | 4,61846117 | 9,617838262 | 0,48019742 |
| 300 | ILMN_1822384 | Hs50756 | 7,2834819 | 1,881806402 | 3,87047354 |

## Claims

1. A method for the detection of active Tuberculosis (hereinafter shortly 'TB') in a human subject based on RNA from a blood sample obtained from said subject, comprising:
measuring the abundance of at least 4 RNAs in the sample, that are chosen from the RNAs listed in Table 1, and
concluding based on the measured abundance whether the subject hasTB.

2. The method of claim 1, wherein the abundance of at least 6 RNAs, of at least 8 RNAs, of at least 12 RNAs, of at least 14 RNAs that are chosen from the RNAs listed in Table 1 is measured.

3. The method of claim 1 or 2, wherein concluding comprises classifying the sample as being from a healthy individual or from an individual having TB based on the specific difference of the abundance of the at least 4 RNAs in healthy individuals versus the abundance of the at least 5 RNAs in individuals with TB.

4. The method of claims 1 to 4, wherein concluding whether the subject has TB comprises the step of training a classification algorithm on a training set of cases and controls, and applying it to measured RNA abundance.

5. The method of claim 3 or 4, wherein the classifying is achieved by applying a random forest method, a support vector machine (SVM), or a K-nearest neighbor method (K-NN), such as a 3-nearest neighbor method (3-NN), linear discimination analysis (LDA) or prediction analysis for microarrays (PAM).

6. The method of claims 1 to 5, wherein the measuring of RNA abundance is performed using a microarray, a real-time polymerase chain reaction or sequencing.

7. The method of claims 1 to 6, wherein the measuring of the abundance is performed through a hybridization with probes for determining the abundance of the at least 4 RNAs of Table 1, preferably said probes comprise 15 to 150, most preferably 30 to 70 consecutive nucleotides with a reverse complementary sequence to the at least 4 RNAs whose abundance is to be determined.

8. The method of claims 1 to 7, wherein
(i) the RNA of the sample to be determined is mRNA, cDNA, micro RNA, small nuclear RNA, unspliced RNA, or its fragments; and/or
(ii) the abundance of the RNAs in the sample are increased or decreased as shown in Table 1.

9. A microarray, comprising a solid support and a set of oligonucleotide probes, the set containing from 4 to about 3,000 probes, and including at least 4 probes for detecting an RNA selected from Table 1.

10. The microarray of claim 9, wherein the probes comprise 15 to 150, preferably 30 to 70 consecutive nucleotides with a reverse complementary sequence to the at least 4 RNAs.

11. Use of a microarray of claim 9 or 10 for detection of TB in a human subject based on RNA from a blood sample, comprising measuring the abundance of at least 4 RNAs listed in Table 1.

12. A kit for the detection of TB in a human subject based on RNA obtained from a blood sample, comprising means for measuring the abundance of at least 4 RNAs that are chosen from the RNAs listed in Table 1, preferably comprising means for exclusively measuring the abundance of RNAs that are chosen from Table 1.

13. The kit of claim 12, which comprises
probes comprising 15 to 150, preferably 30 to 70 consecutive nucleotides with a reverse complementary sequence to the at least 4 RNAs whose abundance is to be determined, or
a microarray comprising probes with a reverse complementary sequence to the at least 4 RNAs whose abundance is to be determined.

14. Use of a kit of claim 12 or 13 for the detection of TB in a human subject based on RNA from a blood sample, comprising means for measuring the abundance of at least 4 RNAs that are chosen from the RNAs listed in Table 1, comprising measuring the abundance of at least 4 RNAs in a blood sample from a human subject, wherein the at least 4 RNAs are chosen from the RNAs listed in Table 1, and concluding based on the measured abundance whether the subject has TB.

15. A method for preparing an RNA expression profile that is indicative of the presence or absence of TB in a subject, comprising: isolating RNA from a blood sample obtained from the subject, and determining the abundance of from 4 to about 3000 RNAs, including at least 4 RNAs selected from Table 1.
